# EUROPEAN PATENT APPLICATION

(11) **EP 4 502 597 A1**
(43) Date of publication of application: **05.02.2025**
(21) Application number: 23779551.3
(22) Date of filing: 14.03.2023
(51) Int. Cl.: G01N 33/18, C02F 1/00, G01D 3/00, G01D 18/00, G08C 25/00, H04Q 9/00

(54) **WATER QUALITY SENSOR CALIBRATION SYSTEM, WATER QUALITY MANAGEMENT SYSTEM, AND WATER QUALITY MANAGEMENT METHOD**

(30) Priority: 29.03.2022 JP 2022054203
(71) Applicant: Hitachi Systems, Ltd., Tokyo 141-8672 (JP)
(72) Inventor: UEKAWA, Kyohei, Tokyo 141-8672 (JP); TAGUCHI,Takuzo, Tokyo 141-8672 (JP); SETOGUCHI,Tadashi, Tokyo 151-0053 (JP)
(74) Representative: Hoffmann Eitle
(86) International application number: PCT/JP2023/009809
(87) International publication number: WO 2023/189531

(57) **Abstract**

To provide a technique capable of facilitating water quality sensor calibration and drainage operations for improving water quality even when water quality monitoring devices and drainage devices are installed in places where operation is difficult, such as inside manholes.

In a case of a deviation between a manual analysis value obtained at a water quality monitoring device (water quality sensor) installation site and the water quality measurement value (e.g., residual chlorine concentration) measured by the water quality monitoring device, a calibration value obtained based on the manual analysis value is stored in a cloud database via a communication terminal (communication unit) and network, and the water quality monitoring device downloads (acquires) this calibration value, and calibrates the water quality sensor based on the calibration value. Additionally, water quality can be improved by remotely managing drainage operations and controlling the start and stop of drainage operations according to the water quality.

## Description

### [Technical Field]

The present invention relates to a water quality sensor calibration system for calibrating a water quality sensor in a water quality monitoring device that is installed in a manhole (for example, a water quality control room or a fire hydrant room) in the terminal regions of a water transportation system and for monitoring the water quality (such as the residual chlorine concentration) of tap water, as well as a water quality management system and a water quality management method for improving water quality.

### [Background of the Invention]

Tap water includes chlorine for disinfection of pathogenic microorganisms. Accordingly, water quality monitoring devices include water quality sensors such as a residual chlorine concentration meters for monitoring chlorine concentration. The concentration of chlorine differs between upstream water purification plants and downstream terminal areas and consumers, and changes over time. For this reason, water utility companies have installed automatic water quality measurement devices on roads and in management facilities to continuously monitor residual chlorine and keep it at an appropriate concentration at all times. However, in the event of a change in the water quality measurement values (such as the residual chlorine concentration) measured by the water quality sensor, it becomes necessary to appropriately calibrate the measurement values measured by the water quality sensor.

Conventionally, operators visited the site where the water quality monitoring device was installed to identify the water quality measurement value (chlorine concentration), connected the operation panel of the water quality monitoring device, personal computer, PLC, and other device terminals to the water quality monitoring device, and carried out the water quality sensor calibration operation.

### [Citation List]

### [Patent Document]

[Patent Document 1] Japanese Patent No. 6547068

### [Summary of Invention]

### [Technical Problem]

However, when calibrating a water quality sensor of a water quality monitoring device installed in a manhole or the like according to the conventional methods, it can be difficult to operate the panels or the like due to the accumulation of sediment and the wet conditions, for instance. In addition, the installation location of water quality monitoring devices often differ depending on the site, and there are problems in that it is difficult for workers to connect equipment such as personal computers to the on-site water quality monitoring equipment. In addition, although Patent Document 1 describes the installation of a water quality sensor in a manhole, calibration of the water quality sensor is not considered.

Accordingly, an object of the present invention is to provide a technique that is capable of facilitating water quality sensor calibration and drainage operations for improving water quality even when water quality monitoring devices and drainage devices are installed in places where operation is difficult, such as inside manholes.

### [Means for Solving the Problems]

In order to solve the above problem, one representative water quality monitoring device, water quality sensor calibration system in the water quality monitoring device, and calibration method according to the present invention relates to storing, in the case that that is an unacceptable deviation between a manual analysis value obtained at the site where the water quality monitoring device is installed and a measured value (a water quality measurement value) measured by the water quality monitoring device, a calibration value obtained based on the manual analysis value in a database of a water quality measurement value management device via a communication unit (a communication terminal) and a network, and downloading (acquiring), by the water quality monitoring device, this calibration value and using the calibration value to calibrate the water quality sensor.

Further, one aspect of the present invention relates to a water quality monitoring device that is installed in a water quality control room and has functionality for transmitting water quality measurement values measured by a water quality sensor to the water quality measurement value management device that manages water quality measurement values, wherein the water quality monitoring device includes a communication unit for accessing the water quality measurement value management device and downloading a calibration value stored in the database of the water quality measurement value management device, and a water quality sensor calibration processing device for receiving the calibration value downloaded by the communication unit and calibrating the water quality measurement value from the water quality sensor based on the calibration value, and calibrating the water quality sensor based on the calibration value downloaded from the water quality measurement value management device.

For example, in a case where there is a deviation between the manual analysis value obtained by manual analysis using a portable residual chlorine measurement device at a site where the water quality monitoring device is installed and a cloud value (the water quality measurement value measured by the water quality monitoring device), a calibration value obtained based on the manual analysis value is entered into a calibration value input unit such as a business-use tablet or a business-use smart phone, communicated to the cloud side via the network, and stored in a database on the cloud. The water quality monitoring device downloads (acquires) the calibration value from the water quality measurement value management device side, applies the calibration value to the water quality sensor, and calibrates the water quality sensor.

Here, it is necessary for the calibration value obtained based on the manual analysis value to be immediately applied to the water quality sensor operating in the field. This is because the water quality value varies with time.

**In** addition, the communication of the calibration value may be performed using a switch having a waterproof function, such as a magnet switch that opens and closes with a magnet. At this time, the water quality measurement value management device determines that the calibration value is stored in the database, and sends the calibration value to the communication unit.

The communication unit is dust-proof and waterproof, and thus does not include an operation panel or buttons. For this reason, a method of performing communication using a magnet switch is utilized, but other methods may be utilized provided that the dust-proof and water-proof specifications are maintained.

### [Advantageous Effects of Invention]

According to the present invention, it is possible to enable facilitation of water quality sensor calibration and drainage operations for improving water quality even when water quality monitoring devices and drainage devices are installed in places where operation is difficult, such as inside manholes, and efficient calibration and drainage control become possible.

Problems, configurations, and effects other than those described above will be made apparent from the following description of embodiments.

### [Brief Description of Drawings]

[Fig. 1] FIG. 1 is a diagram illustrating an overview of a normal state and a calibration state of a water quality sensor calibration system in which a water quality monitoring device is installed on-site.
[Fig. 2] FIG. 2 is a block diagram illustrating a configuration of a calibration processing device applied to the water quality sensor calibration system and the water quality monitoring device of the present invention.
[Fig. 3] FIG. 3 is a flowchart illustrating an embodiment of a processing procedure of the water quality sensor calibration system of the present invention.
[Fig. 4] FIG. 4 is a diagram illustrating an example of the configuration of the water quality management system of the present invention.
[Fig. 5] FIG. 5 is a diagram illustrating a flow of a drainage control process in the water quality management system of the present invention.
[Fig. 6] FIG. 6 is a diagram illustrating a flow of a drainage start process in the water quality management system of the present invention.
[Fig. 7] FIG. 7 is a diagram illustrating a flow of a drainage stop process in the water quality management system of the present invention.
[Fig. 8] FIG. 8 is a diagram illustrating a computer system for implementing the embodiments of the present invention.

### [Description of Embodiment(s)]

Hereinafter, the embodiments will be described with reference to the drawings.

### [Embodiment 1]

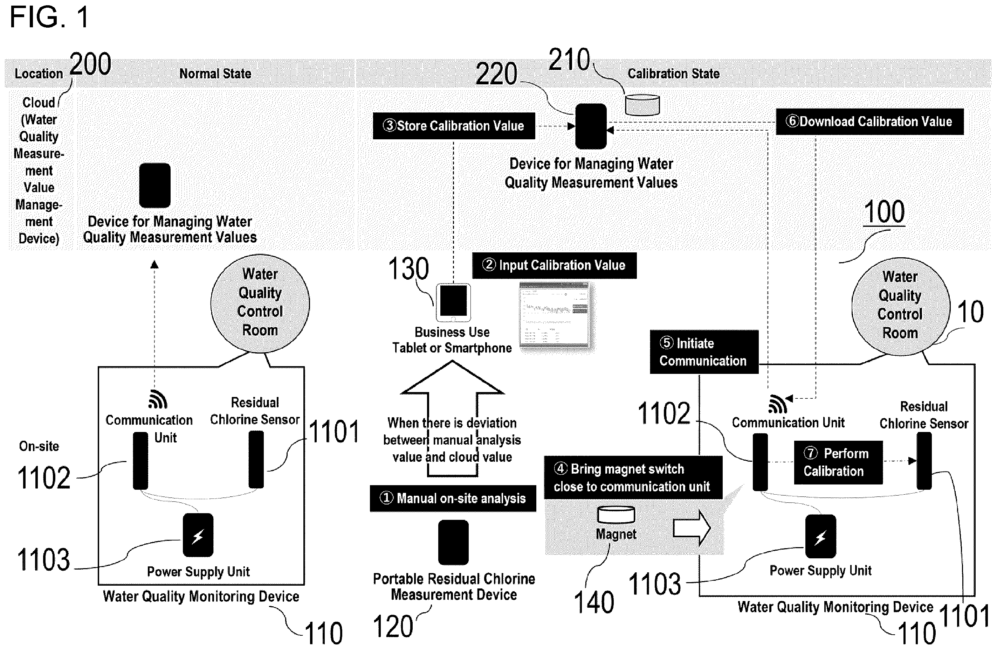

FIG. 1 is a diagram illustrating an overview of a normal state and a calibration state of a water quality sensor calibration system 100 in which a water quality monitoring device 110 is installed on-site. It should be noted that, in embodiments, it is assumed that the chlorine concentration is monitored and calibrated as an indicator of water quality, but calibration of water quality monitoring indicators other than chlorine concentration (for example, chromaticity, turbidity, pH, and the like), or measurement values such as water pressure and flow rate, is also possible.

In the present figure, the water quality sensor calibration system 100 remotely performs calibration of a water quality sensor 1101 (for example, a residual chlorine concentration meter, a chromaticity meter, a turbidity meter, a pH meter, or the like) from the site through the cloud 200 (the water quality measurement value management device 220, the database 210); that is, the water quality sensor calibration system 100 overwrites the water quality measurement values with calibration values (an average value of water quality measurement values measured a plurality of times, such as three times, for example, by the water quality analysis unit 120). The water quality sensor calibration system 100 includes a water quality monitoring device 110 installed on site, a water quality analysis unit 120 for manually analyzing water quality at the site, a calibration value input unit 130 for inputting calibration values for the water quality sensor 1101, a database 210 and a water quality measurement value management device 220. The water quality measurement value management device 220 is implemented, for example, in the cloud 200 or outside the cloud 200, and includes a server, a personal computer, or the like.

The water quality monitoring device 110 is installed in a water quality control room 10 (not illustrated in the figures) of an underground facility, such as a manhole, for example, and includes a water quality sensor 1101 for monitoring water quality, a communication unit 1102 that has communication functionality, and a power supply unit 1103 that supplies power. As will be described later, the water quality monitoring device 110 may be a processing unit (CPU), for example, and includes a water quality sensor calibration processing device 1104 that calibrates a water quality measurement value of the water quality sensor 1101.

Since the water quality monitoring device 110 is installed in an underground facility, it is configured to be dust-proof and water-proof with a protective grade of IP67 or higher.

The water quality sensor 1101 may, for example, include a residual chlorine concentration meter that detects the concentration of chlorine remaining in the water flowing into water pipes.

The communication unit 1102 transmits the residual chlorine concentration value (the water quality measurement value) measured by the residual chlorine concentration meter serving as the water quality sensor 1101 to the cloud 200, and is a terminal that initiates (starts) communication using, for example, a magnet switch (an electromagnetic switch) having functionality for opening and closing a conductive contact when a magnet 140 approaches.

That is, when a manual analyst (operator) brings a magnet close to the communication unit 1102, a magnet switch (not illustrated in the Figures) incorporated in the communication unit 1102 is turned on, and communication is initiated. The communication unit 1102 has functionality for accessing the water quality measurement value management device 220 of the cloud 200, downloading calibration values stored in the database 210 of the cloud 200, and receiving the calibration values, for example.

The power supply unit 1103 supplies power to the water quality sensor 1101 (the residual chlorine detection sensor) and the communication unit 1102.

The water quality analysis unit 120 includes, for example, a portable residual chlorine measurement device, a portable chromaticity measurement device, a portable turbidity measurement device, or a portable pH measurement device. In a case that the manual analysis value (the chlorine concentration value) manually analyzed by the water quality analysis unit 120 at the site deviates from the cloud 200 value, that is, the water quality measurement value (the chlorine concentration value) transmitted from the water quality monitoring device 110 to the cloud 200 via the communication unit during a normal state, the manual analyst obtains the calibration value from the manual analysis value, and inputs the calibration value from the calibration value input unit 130.

Here, the deviation indicates that there is an unacceptable predetermined difference between the numerical values of the manual analysis value and the water quality measurement value, and, as an example, in the case of measuring water quality using residual chlorine concentration, refers to a case where the manual analysis value and the water quality measurement value are separated by±0.03mg/L to ±0.05mg/L or more. However, this deviation, that is, the predetermined difference, may be set according to the past operational experience of the individual monitoring the water quality and the monitoring location, and may vary depending on the water utility company (such as the municipal water supply bureau), for example. Accordingly, it is not necessary to uniquely specify the difference between the numerical values, and this difference may be set in accordance with the operation of the individual who monitors the water quality.

In addition, the calibration value may be obtained by the methods of each water utility company. For example, in a case that the water quality sensor 1101 performs measurement of the water quality a plurality of times, such as three times, the average value of the three water quality measurement values may be obtained as the calibration value.

The calibration value input unit 130 includes, for example, a terminal such as a business-use tablet or a business-use smartphone, and has functionality for inputting a calibration value obtained from an analysis value manually analyzed by a portable residual chlorine measurement device and communicating the calibration value to the cloud 200 via a network 300 (the Internet or the like).

The cloud 200 includes a database 210 that stores the water quality measurement values of the water quality sensor 1101 transmitted from the communication unit 1102 and the calibration values transmitted from the calibration value input unit 130, and a water quality measurement value management device 220 that manages these water quality measurement values (including the calibration values).

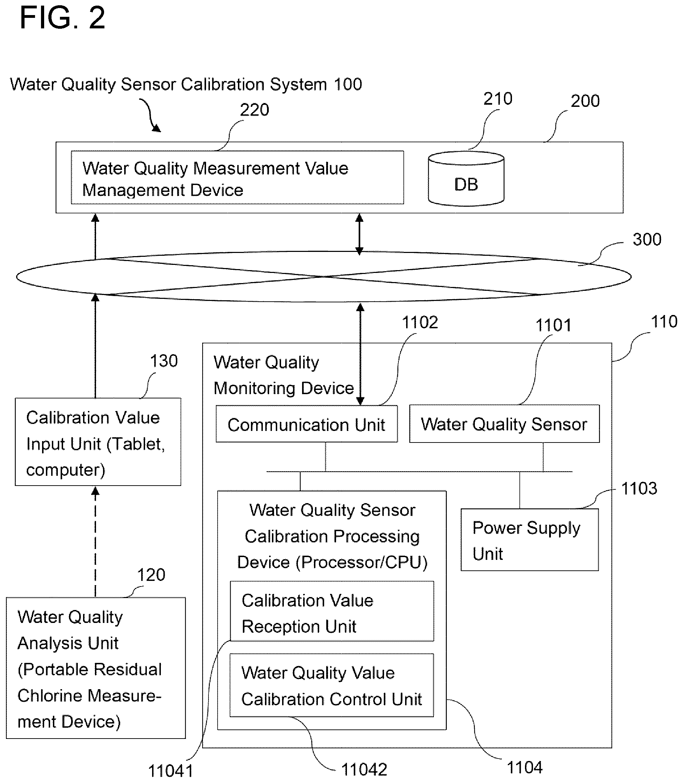

FIG. 2 is a block diagram illustrating a configuration of a water quality sensor calibration processing device 1104 applied to the water quality sensor calibration system 100 and the water quality monitoring device 110 of the present invention.

The water quality sensor calibration processing device 1104 includes a processing device (CPU), and has functionality for calibrating the water quality sensor 1101 based on a calibration value from the cloud 200.

The processing device includes a calibration value reception unit 11041 and a water quality value calibration control unit 11042 for receiving the calibration values from the communication unit 1102. The water quality value calibration control unit 11042 calibrates the water quality sensor 1101 based on the calibration value so that the water quality measurement value measured by the water quality sensor 1101 becomes the calibration value obtained from the result of the manual analysis value manually analyzed by the water quality analysis unit 120.

Next, the water quality calibration of the water quality measurement value measured by the water quality sensor 1101 is performed by the following operation procedure.

### First:

(1) The manual analyst brings the portable residual chlorine measurement device, which serves as the water quality analysis unit 120, to the site and performs manual analysis of the water quality.
(2) When there is a deviation between the analyzed value and the measured water quality value (the residual chlorine concentration value) on the cloud 200 side, the manual analyst obtains a calibration value based on the manual analysis value and inputs the calibration value to the calibration value input unit 130. The calibration value input unit 130 transmits the input calibration value to the cloud 200.
(3) The cloud 200 stores the calibration value transmitted from the calibration value input unit 130 in the database 210.

### Next:

(4) The manual analyst brings the magnet 140 close to the communication unit 1102 and activates the magnet switch of the communication unit 1102.

(5) As a result, the communication unit 1102 initiates communication, accesses the cloud 200, and downloads the calibration value.

(6) At this time, the cloud 200 determines whether a calibration value is stored in the database 210, and in a case that a calibration value is stored, transmits the calibration value from the database 210 to the communication unit 1102.

(7) The communication unit 1102 downloads (receives) the calibration value from the cloud 200, and supplies the downloaded calibration value to the water quality sensor calibration processing device 1104 of the water quality monitoring device 110. Then, the water quality sensor calibration processing device 1104 of the water quality monitoring device 110 calibrates the water quality sensor 1101 based on the downloaded calibration value.

That is, when the downloaded calibration value is received, the water quality sensor calibration processing device 1104 overwrites (calibration processing) the water quality measurement value (the residual chlorine concentration value) measured by the water quality sensor 1101 (the residual chlorine concentration meter) with the calibration value determined from the result of the manual analysis, and records the calibration value as the latest water quality measurement value (the residual chlorine value) in the residual chlorine concentration meter. This means that the water quality measurement value measured by the residual chlorine detection sensor is calibrated to become the calibration value determined from the manual analysis value result; that is, the water quality measurement value is overwritten by the calibration value.

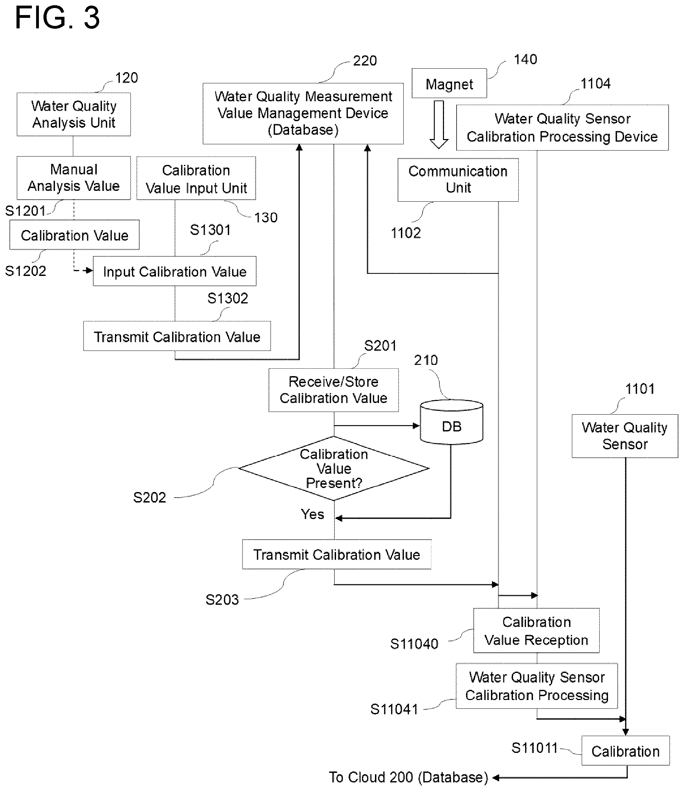

FIG. 3 is a flowchart illustrating an embodiment of a processing procedure of the water quality sensor calibration system 100 of the present invention. The operations based on the flowchart are as follows.

Step S1201: The portable residual chlorine measurement device, which serves as the water quality analysis unit 120, manually analyzes the water quality at the site where the water quality monitoring device 110 is installed.

Step S1202: In the case that the analysis value analyzed by the portable residual chlorine measurement device serving as the water quality analysis unit 120 deviates from the value in the cloud 200 (a water quality measurement value such as a residual chlorine concentration measured by the water quality sensor 1101 of the water quality monitoring device 110), a calibration value is obtained based on the analysis value.

Step S1301: The calibration value input unit 130 inputs the calibration value obtained in Step S1202.

Step S1302: The calibration value input unit 130 transmits the calibration value input in Step S1201 to the cloud 200.

Step S201: The cloud 200 receives the calibration value transmitted in Step S1302 and stores the calibration value in the database 210.

Step S202: The cloud 200 determines the presence or absence of a calibration value in the database 210.

Step S203: Based on the results of the determination in Step S202, in the case that a calibration value is present, the cloud 200 transmits the calibration value to the water quality sensor calibration processing device 1104 via the communication unit 1102. The water quality sensor calibration processing device 1104 receives the calibration value at Step S11040.

Step S11041: The water quality sensor calibration processing device 1104 performs a process of calibrating the water quality sensor 1101 based on the calibration value from the cloud 200.

Step S11011: The water quality sensor 1101 is calibrated by the calibration value from the cloud 200.

According to the above-described embodiment, the above-described effects can be achieved, and reduction of the on-site installation equipment, reduction of the customer installation cost associated therewith, reduction of equipment failure response, the effective use of the installation space, and effective use of the cloud can be achieved.

**In** addition, the water quality calibration system does not need to be equipped with an operation/display panel, buttons, or the like, and the calibration operation of the water quality sensor does not need to be performed by using a panel, a personal computer, or the like in the water quality control room 10 in the manhole.

**In** addition, the realization of a remote system for small water distribution pipes and the realization of water quality monitoring at the terminus of water pipes; that is, the realization of timely drainage operations as a result of efficient concentration determination and operation automation, and further, a reduction in the amount of chlorine agents input in water reservoirs and a reduction in cost, can be made possible. **In** addition, water quality (residual chlorine concentration) measurement and field work automation can also be made possible.

As described above, according to the water quality sensor calibration system and the water quality management method according to the first embodiment of the present disclosure as described with reference to FIG. 1 to FIG. 3, it is possible to facilitate performance of water quality sensor calibration even when water quality monitoring devices are installed in places where operation is difficult, such as inside manholes.

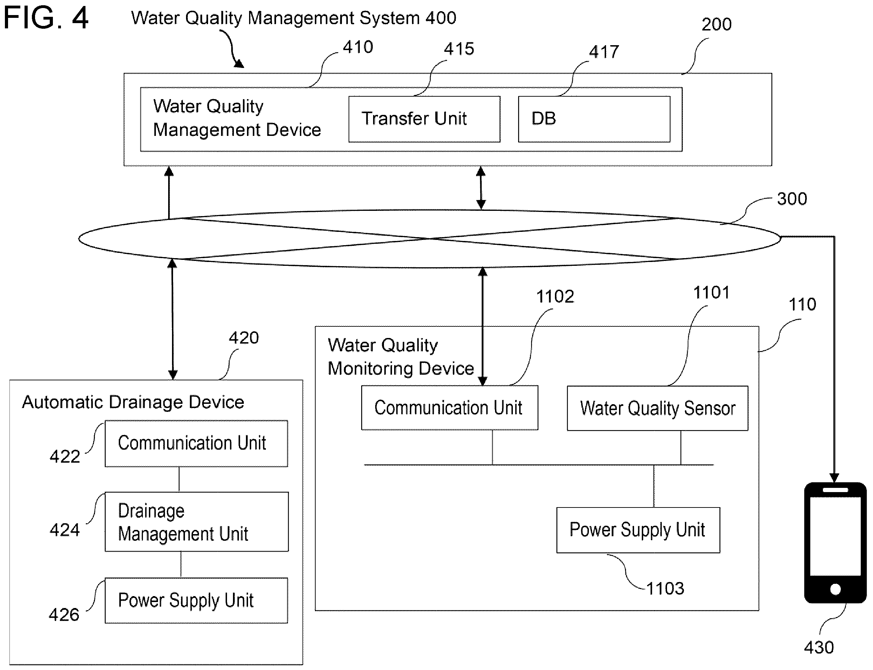

Hereinafter, a water quality management system according to a second embodiment of the present disclosure will be described with reference to FIG. 4 to FIG. 8.

### [Embodiment 2]

As described above, under the provisions of water supply laws, tap water is required to be disinfected with chlorine in order to prevent pathogens and the like from occurring. As such, water utility companies conduct continuous monitoring of residual chlorine and perform management so that chlorine is maintained at an appropriate concentration.

However, in terminal water distribution areas where water consumption is low, water tends to stagnate, and residual chlorine may decrease. This decrease in residual chlorine tends to increase particularly in old water pipes and in summertime when the water temperature of tap water increases
because the decomposition of sodium hypochlorite and the like accelerates.

In this way, when a decrease in the water quality occurs due to a decrease in the residual chlorine concentration, for example, the water quality can be improved by performing an operation of draining the stagnant water. However, in drainage operations, since it is often necessary to go to each site and open and close valves for each location, and since operations are performed for draining water at a fixed time using a timer or during normal operation, it is difficult to control the start and stop of drainage operations automatically in accordance with the condition of the water quality of the site.

Accordingly, the second embodiment of the present disclosure relates to a water quality management system capable of remotely managing a drainage operation and automatically controlling starting and stopping of the drainage operation according to the water quality, for example.

First, with reference to FIG. 4, a configuration of a water quality management system according to the second embodiment of the present disclosure will be described.

FIG. 4 is a diagram illustrating an example of the configuration of the water quality management system 400 according to the second embodiment of the present disclosure. As illustrated in FIG. 4, the water quality management system 400 according to the second embodiment of the present disclosure primarily includes a water quality management device 410, an automatic drainage device 420, a water quality monitoring device 110, and a user terminal 430. The water quality management device 410, the automatic drainage device 420, the water quality monitoring device 110, and the user terminal 430 may be communicably connected via the network 300.

It should be noted that here, in the description of the water quality management system 400, the description of configurations substantially shared with the water quality sensor calibration system 100 described with reference to FIG. 2 will be omitted, and the description will focus on the configuration that differs from the water quality sensor calibration system 100.

The water quality management device 410 is a device for performing information communication with and coordinating the automatic drainage device 420, the water quality monitoring device 110, and the user terminal 430. As will be described later, the water quality management device 410 can control the drainage operation by the automatic drainage device 420 based on instructions received from the water quality monitoring device 110 or the user terminal 430. The water quality management device 410 may be arranged in the cloud 200 or outside the cloud 200, and may be implemented by a server, a personal computer, or the like.

The transfer unit 415 in the water quality management device 410 is a functional unit for performing information communication with the water quality monitoring device 110 and the user terminal 430 via the network 300.

The database 417 in the water quality management device 410 is, for example, a database for storing the various data transmitted from the user terminal 430, such as the starting time and the stopping time of the drainage operation.

The automatic drainage device 420 is a device for performing a drainage operation. More specifically, the automatic drainage device 420 is installed in a drainage chamber, which may be an underground facility that is the same as or different from the water quality control room in which the water quality monitoring device 110 is installed, for example, and can control the start and stop of drainage of stagnant water.

As illustrated in FIG. 4, the automatic drainage device 420 may include a communication unit 422, a drainage management unit 424, and a power supply unit 426.

The power supply unit 426 is a functional unit for supplying power to the communication unit 422 and the drainage management unit 424.

The drainage management unit 424 is a functional unit for starting and stopping the drainage operations of stagnant water based on instructions received from the water quality management device 410. In some embodiments, the drainage management unit 424 may control drainage operations using an automatic on-off valve box that includes an automatic on-off valve.

The communication unit 422 (also referred to as a first communication unit) is a functional unit for performing information communication with the water quality management device 410 via the network 300. In an embodiment, similarly to the communication unit 1102 (also referred to as a second communication unit) of the water quality monitoring device 110 described above, the communication unit 422 may be a terminal that initiates (starts) communication using, for example, a magnet switch (an electromagnetic switch) having functionality for opening and closing a conductive contact when a magnet approaches. In this case, when an operator brings a magnet close to the communication unit 422, a magnet switch (not illustrated in the Figures) incorporated in the communication unit 422 is turned on, and communication is initiated. Then, the communication unit 422 may access the water quality management device 410 of the cloud 200, download information and instructions regarding the date and time to perform drainage operations from the database 417, and receive this information.

The user terminal 430 is a terminal used by a user who manages the automatic drainage device. For example, the user terminal 430 may include a terminal such as a business-use tablet or a business-use smartphone, and after receiving input from a user of a start time and a stop time for a drainage operation, the user terminal 430 may transmit a drainage operation setting request that includes the information on the start time and the stop time of the drainage operation to the water quality management device 410 via the network 300 (the Internet or the like).

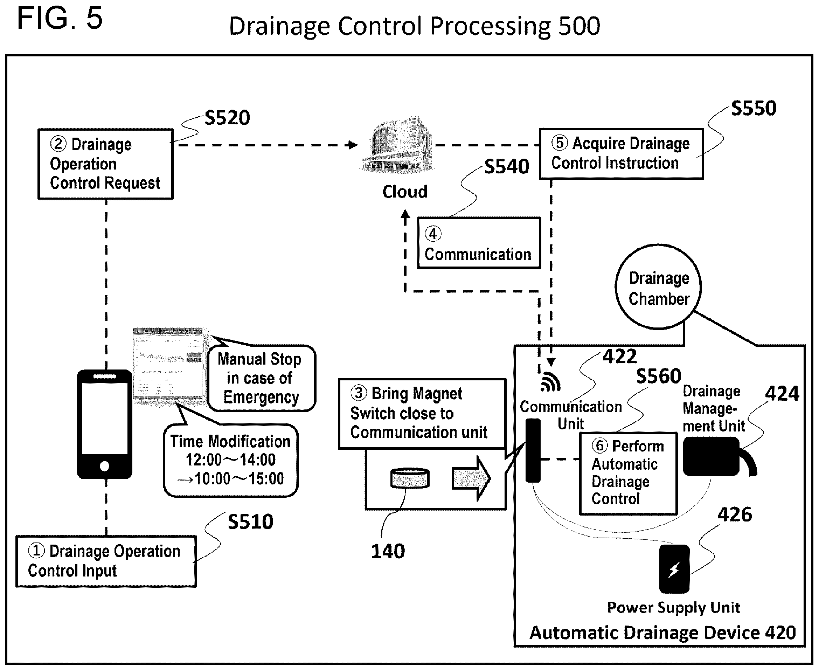

Next, with reference to FIG. 5 to FIG. 7, the flow of operations performed by the water quality management system according to the second embodiment of the present disclosure will be described.

FIG. 5 is a diagram illustrating the flow of a drainage control process 500 for controlling a drainage operation performed by the automatic drainage device 420 based on an instruction from a user in the water quality management system 400 according to the second embodiment of the present disclosure. The drainage control process 500 illustrated in FIG. 5 may be performed by each device or functional unit of the water quality management system 400 described with reference to FIG. 4.

First, in Step S510, a user such as a manual analyst (operator) makes an input to the user terminal 430 specifying a particular control (hereinafter referred to as "drainage operation control") regarding the drainage operations performed by the automatic drainage device 420. The drainage operation control may include, for example, control for changing the date and time of a drainage operation performed periodically by the automatic drainage device 420, control for starting a drainage operation, control for stopping a drainage operation, or the like. As an example, in a case in which a drainage operation is performed daily between 12:00 and 14:00, and a user determines that a longer drainage is necessary to improve water quality, the user may input drainage operation control for changing the execution time of the drainage operation to "10:00 to 15:00."

Here, the drainage operation control may be determined in accordance with the water quality and the circumstances of the surrounding environment. For example, in a case that it is determined that the manual analysis value of the water quality manually analyzed by the user on-site does not satisfy a predetermined water quality standard, a drainage operation control for improving the water quality may be determined and input. In addition, in a case where the occurrence of a natural disaster is predicted or detected, the user may perform an input for defining a drainage operation control for stopping drainage operations in order to save water.

Next, at Step S520, the user terminal 430 transmits, via the network 300, a drainage operation control request to the water quality management device 410 arranged in the cloud 200 requesting the execution of the drainage operation control input by the user in Step S510. The water quality management device 410 that receives the drainage operation control request from the user terminal 430 stores the information of the drainage operation control request in the database 417.

Next, at Step S530, after transmitting the drainage operation control request to the water quality management device 410, the user brings the magnet 140 close to the communication unit 422 and activates the magnet switch of the communication unit 422.

Next, at Step S540, the communication unit 422 in which the magnet switch was activated starts communication with the cloud 200, and at Step S550, the drainage operation control request is downloaded from the database 417.

Next, at Step S560, the drainage management unit 424 of the automatic drainage device 420 performs the particular drainage operation control in accordance with the information of the drainage operation control request acquired in Step S540. Here, the drainage management unit 424 may control the execution of the drainage operation using an automatic opening/closing valve box including one or more automatic opening/closing valves.

According to the drainage control process 500 described above, it is possible to efficiently control the drainage operations performed by the automatic drainage device even in locations in which operation is difficult, such as inside manholes.

It should be noted that, although a case has been described above as an example in which the communication unit 422 includes a magnet switch and is activated when a magnet approaches, the present disclosure is not limited hereto, and the communication unit 422 does not need to include a magnet switch. For example, the communication unit 422 may communicate with the automatic drainage device 420 at a predetermined, periodic communication time.

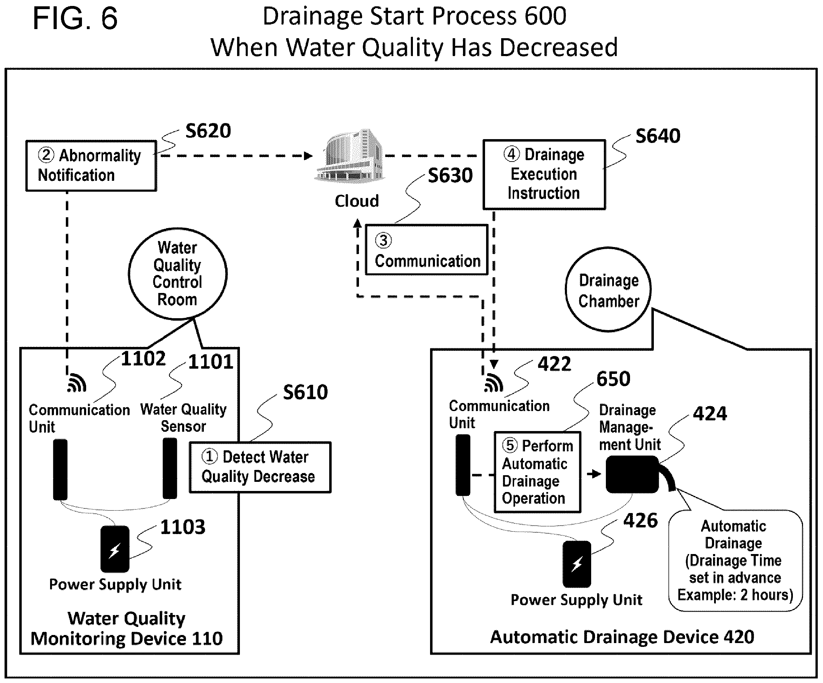

FIG. 6 is a diagram illustrating the flow of a drainage start process 600 for starting a drainage operation using the automatic drainage device 420 when a decrease in water quality is detected in the water quality management system 400 according to the second embodiment of the present disclosure. The drainage start process 600 illustrated in FIG. 6 may be performed by each device or functional unit of the water quality management system 400 described with reference to FIG. 4.

First, in Step S610, the water quality sensor 1101 of the water quality monitoring device 110 measures the water quality and acquires a water quality measurement value. As described above, this measured water quality value may be any value that is an indicator of water quality, such as, for example, residual chlorine concentration, chromaticity, turbidity, or pH. Subsequently, the water quality monitoring device 110 determines whether or not the obtained water quality measurement value satisfies a predetermined water quality criterion. The predetermined water quality criterion may be a predetermined water quality value for evaluating water quality, or may be an appropriate water quality range defined by a lower limit and an upper limit.

As an example, in the case that residual chlorine concentration is used as the water quality value, the water quality monitoring device 110 may determine that the current water quality does not satisfy the predetermined water quality criterion in a case that it is determined that the acquired residual chlorine concentration is lower than the lower limit of the appropriate residual chlorine concentration. Here, the predetermined water quality criterion may be, for example, within a range of ± 0.03mg/L to ±0.05mg/L.

Next, at Step S620, in the case that the communication unit 1102 of the water quality monitoring device 110 determines that the water quality measurement value acquired in Step S610 does not satisfy the predetermined water quality criterion, it transmits an abnormality notification indicating that the water quality has deteriorated to the water quality management device 410 arranged in the cloud 200 via the network 300. The water quality management device 410 which receives the abnormality notification from the water quality monitoring device 110 stores the information of the abnormality notification in the database 417.

Next, in Step S630, the communication unit 422 of the automatic drainage device 420 communicates with the water quality management device 410.

Here, the communication unit 422 may be a magnet switch that is activated when a magnet is brought close. In this case, for example, a user such as an operator who analyzes the water quality on-site can perform the drainage operation in real time on the spot by bringing the magnet close to the communication unit 422 and initiating communication with the automatic drainage device 420.

However, the present disclosure is not limited thereto, and the communication unit 422 may communicate with the automatic drainage device 420 at a predetermined periodic communication time (every hour, every six hours, every day) without using a magnet switch, for example. In this way, even when there is no worker on-site, for example, the drainage operation can be easily controlled.

Next, at S640, the transfer unit 415 of the water quality management device 410 transmits, via the network 300, a drainage execution instruction to the automatic drainage device 420 requesting that the drainage operation be performed at a particular date and time. The drainage execution instruction may be information requesting only the start of the drainage operation, or may be information requesting that the drainage operation be performed at a preset execution time (for example, "10:00 to 15:00").

Next, at Step S650, the drainage management unit 424 of the automatic drainage device 420 performs the drainage operation according to the information of the drainage execution instruction acquired in Step S640. Here, the drainage management unit 424 may control the execution of the drainage operation by using an automatic opening/closing valve box including an automatic opening/closing valve.

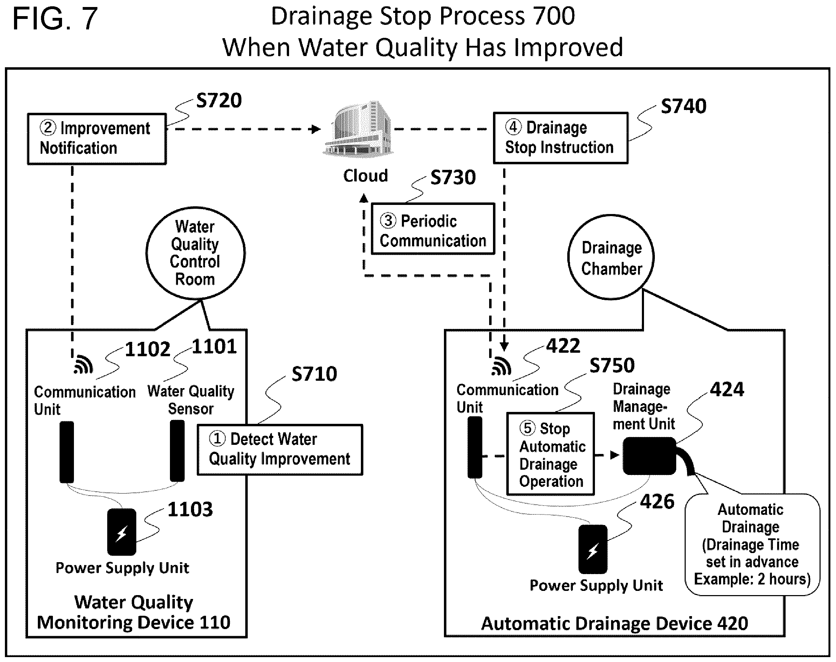

FIG. 7 is a diagram illustrating the flow of a drainage stop process 700 for stopping a drainage operation using the automatic drainage device 420 when an improvement in water quality is detected in the water quality management system 400 according to the second embodiment of the present disclosure. The drainage stop process 700 illustrated in FIG. 7 is a process performed following the drainage start process 600 described with reference to FIG. 6, and may be performed by each device or functional unit of the water quality management system 400 described with reference to FIG. 4.

In the following description, description that is redundant with the drainage start process 600 described with reference to FIG. 6 will be omitted.

First, at Step S710, the water quality sensor 1101 of the water quality monitoring device 110 measures the water quality and acquires a water quality measurement value (also referred to as a second water quality measurement value). Subsequently, the water quality monitoring device 110 determines whether or not the obtained water quality measurement value satisfies a predetermined water quality criterion.

As an example, in the case that residual chlorine concentration is used as the water quality value, the water quality monitoring device 110 may determine that the current water quality satisfies a predetermined water quality criterion when the acquired residual chlorine concentration is equal to or higher than the lower limit of the appropriate residual chlorine concentration.

Next, at Step S720, in the case that the communication unit 1102 of the water quality monitoring device 110 determines that the water quality measurement value acquired in Step S710 satisfies the predetermined water quality standard, it transmits an improvement notification indicating that the water quality has improved to the water quality management device 410 arranged in the cloud 200 via the network 300. The water quality management device 410 that receives the improvement notification from the water quality monitoring device 110 stores the information of the improvement notification in the database 417.

Next, at Step S730, the communication unit 422 of the automatic drainage device 420 communicates with the water quality management device 410.

Next, at Step S740, the transfer unit 415 of the water quality management device 410 transmits, via the network 300, a drainage stop instruction to the automatic drainage device 420 requesting that the drainage operation be stopped at a predetermined date and time. The drainage stop instruction may be information requesting an immediate stop of the drainage operation, or may be information requesting that the drainage be performed at a preset stop time (for example, "15:00").

Next, at Step S750, the drainage management unit 424 of the automatic drainage device 420 stops the drainage operation in accordance with the information of the drainage stop instruction acquired in Step S740. Here, the drainage management unit 424 may control the execution of the drainage operation by using an automatic opening/closing valve box including an automatic opening/closing valve.

According to the water quality management system 400 according to the second embodiment of the present disclosure described above, it is possible to remotely manage drainage operations and automatically control starting and stopping of drainage operations. The control of drainage operations may be performed according to, for example, a prediction of a natural disaster, a change in water quality, or the like. For example, in a case where a decrease in water quality is detected, it is possible to remotely manage control for stopping a drainage operation when an improvement in water quality is detected after a drainage operation is started, control for changing the execution time of periodically performed drainage operations, and the like.

In this way, it is possible to easily perform drainage operations for improving water quality, such as drainage operations which could only be done locally or more meticulous drainage operations according to local water quality.

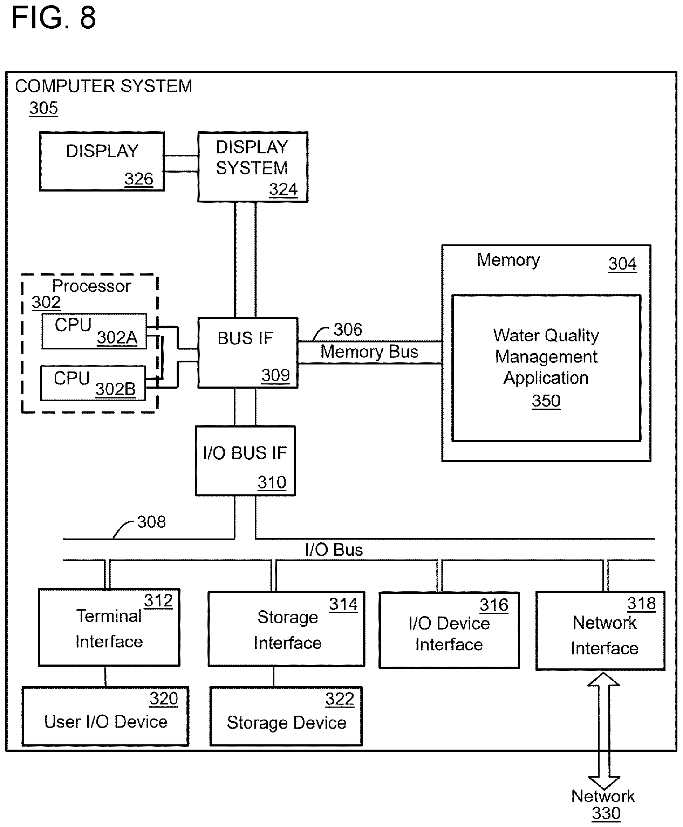

With reference to FIG. 8, a computer system 305 for implementing the embodiments of the present disclosure will be described. The mechanisms and devices of the various embodiments disclosed herein may be applied to any suitable computing system. The main components of the computer system 305 include one or more processors 302, a memory 304, a terminal interface 312, a storage interface 314, an I/O (Input/Output) device interface 316, and a network interface 318. These components may be interconnected via a memory bus 306, an I/O bus 308, a bus interface unit 309, and an I/O bus interface unit 310.

The computer system 305 may include one or more general purpose programmable central processing units (CPUs), 302A and 302B, herein collectively referred to as the processor 302. In some embodiments, the computer system 305 may contain multiple processors, and in other embodiments, the computer system 305 may be a single CPU system. Each processor 302 executes instructions stored in the memory 304 and may include an on-board cache.

In some embodiments, the memory 304 may include a random access semiconductor memory, storage device, or storage medium (either volatile or non-volatile) for storing data and programs. The memory 304 may store all or a part of the programs, modules, and data structures that perform the functions described herein. For example, the memory 304 may store a water quality management application 350. In some embodiments, the water quality management application 350 may include instructions or statements that execute the functions described below on the processor 302.

In some embodiments, the water quality management application 350 may be implemented in hardware via semiconductor devices, chips, logic gates, circuits, circuit cards, and/or other physical hardware devices in lieu of, or in addition to processor-based systems. In some embodiments, the water quality management application 350 may include data other than instructions or statements. In some embodiments, a camera, sensor, or other data input device (not shown) may be provided to communicate directly with the bus interface unit 309, the processor 302, or other hardware of the computer system 305.

The computer system 305 may include a bus interface unit 309 for communicating between the processor 302, the memory 304, a display system 324, and the I/O bus interface unit 310. The I/O bus interface unit 310 may be coupled with the I/O bus 308 for transferring data to and from the various I/O units. The I/O bus interface unit 310 may communicate with a plurality of I/O interface units 312, 314, 316, and 318, also known as I/O processors (IOPs) or I/O adapters (IOAs), via the I/O bus 308.

The display system 324 may include a display controller, a display memory, or both. The display controller may provide video, audio, or both types of data to the display device 326. Further, the computer system 305 may also include a device, such as one or more sensors, configured to collect data and provide the data to the processor 302.

For example, the computer system 305 may include biometric sensors that collect heart rate data, stress level data, and the like, environmental sensors that collect humidity data, temperature data, pressure data, and the like, and motion sensors that collect acceleration data, movement data, and the like. Other types of sensors may be used. The display system 324 may be connected to a display device 326, such as a single display screen, television, tablet, or portable device.

The I/O interface unit is capable of communicating with a variety of storage and I/O devices. For example, the terminal interface unit 312 supports the attachment of a user I/O device 320, which may include user output devices such as a video display device, a speaker, a television or the like, and user input devices such as a keyboard, mouse, keypad, touchpad, trackball, buttons, light pens, or other pointing devices or the like. A user may use the user interface to operate the user input device to input input data and instructions to the user I/O device 320 and the computer system 305 and receive output data from the computer system 305. The user interface may be presented via the user I/O device 320, such as displayed on a display device, played via a speaker, or printed via a printer.

The storage interface 314 supports the attachment of one or more disk drives or direct access storage devices 322 (which are typically magnetic disk drive storage devices, but may be arrays of disk drives or other storage devices configured to appear as a single disk drive). In some embodiments, the storage device 322 may be implemented as any secondary storage device. The contents of the memory 304 are stored in the storage device 322 and may be read from the storage device 322 as needed. The I/O device interface 316 may provide an interface to other I/O devices such as printers, fax machines, and the like. The network interface 318 may provide a communication path so that computer system 305 and other devices can communicate with each other. The communication path may be, for example, the network 330.

In some embodiments, the computer system 305 may be a multi-user mainframe computer system, a single user system, or a server computer or the like that has no direct user interface and receives requests from other computer systems (clients). In other embodiments, the computer system 305 may be a desktop computer, a portable computer, a notebook computer, a tablet computer, a pocket computer, a telephone, a smart phone, or any other suitable electronic device.

### [Reference Signs List]

100... Water quality sensor calibration system
110... Water quality monitoring device
1101... Water quality sensor
1102, 422. Communication unit
1103, 426... Power supply unit
1104... Water quality sensor calibration processing device
11041... Calibration value receiving unit
11042... Water quality value calibration control unit
120... Water quality analysis unit
130... Calibration value input unit
200... Cloud
210, 417... Database
220... Water quality measurement value management device
400... Water quality management system
415... Transfer unit
420... Automatic drainage device
424... Drainage management unit
430... User terminal

## Claims

1. A water quality sensor calibration system comprising:
a water quality measurement value management device;
a water quality monitoring device;
a water quality analysis unit; and
a calibration value input unit,
wherein:
the water quality measurement value management device manages water quality measurement values,
the water quality monitoring device is installed in a water quality control room, includes a communication unit, a water quality sensor calibration processing device, and a water quality sensor, and has functionality for transmitting a water quality measurement value measured by the water quality sensor to the water quality measurement value management device;
the water quality analysis unit has functionality for manually analyzing water quality at a site where the water quality monitoring device is installed;
in a case that there is an unacceptable deviation between a manual analysis value manually analyzed by the water quality analysis unit and the water quality measurement value managed by the water quality measurement value management device:
the calibration value input unit inputs a calibration value for calibrating the water quality measurement value obtained based on the manual analysis value and transmits the calibration value to the water quality measurement value management device; and
the communication unit accesses the water quality measurement value management device and downloads the calibration value stored in a database of the water quality measurement value management device;
the water quality sensor calibration processing device receives the calibration value downloaded by the communication unit, calibrates the water quality measurement value from the water quality sensor based on the calibration value, and remotely calibrates the water quality sensor based on the calibration value downloaded from the water quality measurement value management device.

2. The water quality sensor calibration system according to claim 1, wherein the water quality sensor calibration processing device includes:
a water quality value calibration control unit having functionality for calibrating, in a case that there is a deviation between the manual analysis value manually analyzed by the water quality analysis unit and the water quality measurement value managed by the water quality measurement value management device, the water quality measurement value measured by the water quality sensor to the calibration value obtained from the manual analysis value based on the calibration value obtained based on the manual analysis value.

3. The water quality sensor calibration system according to either of claim 1 or 2, wherein:
the water quality sensor is any one selected from the group consisting of a residual chlorine concentration meter, a chromaticity meter, a turbidity meter, and a pH meter;
the water quality analysis unit is any one selected from the group consisting of a portable residual chlorine measurement device, a portable chromaticity measurement device, a portable turbidity measurement device, and a portable pH measurement device;
the water quality measurement value management device is any one selected from the group consisting of a cloud-side server and a personal computer;
the calibration value input unit is a terminal including a tablet or a smartphone;
the communication unit is a communication terminal that includes a magnet switch, and is configured such that when a magnet is brought close to the magnet switch after the calibration value is transmitted from the calibration value input unit to the cloud, the magnet switch activates to initiate communication, and the communication unit accesses the water quality measurement value management device and downloads the calibration value stored in the database; and
the water quality sensor calibration processing device is a processing device that calibrates the water quality sensor based on the calibration value.

4. A water quality management system comprising:
an automatic drainage device installed in a drainage chamber to manage a drainage operation;
a user terminal for use by a user; and
a water quality management device for remotely managing the automatic drainage device,
wherein:
the automatic drainage device, the user terminal, and the water quality management device are connected via a communication network;
the water quality management device includes:
a transfer unit configured to transfer, in a case that a drainage operation control request that designates drainage control for starting or stopping a drainage operation at a particular date and time is received from the user terminal, a drainage control instruction to the automatic drainage device that requests that the drainage control be performed at the particular date and time, and
the automatic drainage device includes:
a first communication unit configured to receive communication from the water quality management device, and
a drainage management unit configured to execute, in a case that the drainage control instruction is received from the water quality management device via the first communication unit, the drainage control at the particular date and time.

5. The water quality management system according to claim 4, further comprising a water quality monitoring device installed in a water quality control room, wherein:
the water quality monitoring device includes:
a water quality sensor that acquires a water quality measurement value at a site where the water quality monitoring device is installed, and
a second communication unit that transmits, in a case that the water quality measurement value does not meet a predetermined water quality criterion, an abnormality notification to the water quality management device;
the water quality management device transmits, using the transfer unit, in a case that the abnormality notification is received from the water quality monitoring device, a drainage execution instruction to the automatic drainage device that requests starting of a drainage operation; and
the automatic drainage device starts, in a case that the drainage execution instruction is received from the water quality management device by the first communication unit, the drainage operation.

6. The water quality management system according to claim 5, wherein:
the water quality monitoring device transmits, in a case that a second water quality measurement value measured by the water quality sensor after the abnormality notification was transmitted to the water quality management device satisfies the predetermined water quality criterion, a water quality improvement notification to the water quality management device via the second communication unit that indicates that water quality has improved;
the water quality management device transmits, in a case that the water quality improvement notification is received from the water quality monitoring device, a drainage stop instruction to the automatic drainage device using the transfer unit that requests stoppage of the drainage operation; and
the automatic drainage device stops, in a case that the drainage stop instruction is received from the water quality management device by the first communication unit, the drainage operation.

7. The water quality management system according to claim 4, wherein the first communication unit includes a magnet switch, and is configured such that when a magnet is brought close to the magnet switch, the magnet switch activates to initiate communication, and the first communication unit accesses the water quality management device and acquires information regarding the particular date and time for performing the drainage operation.

8. A water quality management method for a water quality management system that includes at least a water quality monitoring device that is installed in a water quality control room and that has functionality for transmitting a water quality measurement value measured by a water quality sensor to a water quality measurement value management device for managing water quality measurement values, the water quality management method comprising steps that cause the water quality monitoring device to perform:
a water quality analysis step of manually analyzing water quality at a site where the water quality monitoring device is installed;
a calibration value input step of inputting, in a case that there is an unacceptable deviation between a manual analysis value manually analyzed in the water quality analysis step and a water quality measurement value managed by the water quality measurement value management device, a calibration value for calibrating the water quality measurement value obtained based on the manual analysis value and transmitting the calibration value to the water quality measurement value management device;
a communication step of accessing the water quality measurement value management device and downloading the calibration value stored in a database of the water quality measurement value management device;
a water quality sensor calibration processing step of receiving the calibration value downloaded in the communication step and calibrating the water quality measurement value of the water quality sensor based on the calibration value; and
a remote calibration step of remotely calibrating the water quality sensor based on the calibration value downloaded from the water quality measurement value management device.

9. The water quality management method according to claim 8, wherein:
the water quality management system further includes:
an automatic drainage device installed in a drainage chamber to manage a drainage operation;
a user terminal for use by a user; and
a water quality management device for remotely managing the automatic drainage device, and
the water quality management method further includes:
a drainage control instruction step of transferring, in a case that a drainage operation control request that designates drainage control for starting or stopping a drainage operation at a particular date and time is received from the user terminal, a drainage control instruction from the water quality management device to the automatic drainage device that requests that the drainage control be performed at the particular date and time, and
a drainage operation control step of performing, in a case that the automatic drainage device receives the drain control instruction from the water quality management device, the drainage control at the particular date and time.

10. The water quality management method according to claim 9, further comprising:
an abnormality notification step of transmitting, in a case that the water quality measurement value measured by the water quality sensor of the water quality monitoring device does not satisfy a predetermined water quality criterion, an abnormality notification from the water quality monitoring device to the water quality management device;
a drainage execution instruction step of transmitting, in a case that the water quality management device receives the abnormality notification from the water quality monitoring device, a drainage execution instruction to the automatic drainage device that requests starting of a drainage operation; and
a drainage operation start step of starting, in a case that the automatic drainage device receives the drainage execution instruction from the water quality management device, the drainage operation.

11. The water quality management method according to claim 10, further comprising:
an improvement notification step of transmitting, in a case that a second water quality measurement value measured by the water quality sensor after the abnormality notification was transmitted to the water quality management device satisfies the predetermined water quality criterion, a water quality improvement notification that indicates that water quality has improved from the water quality monitoring device to the water quality management device;
a drainage stop instruction step of transmitting, in a case that the water quality management device receives the water quality improvement notification from the water quality monitoring device, a drainage stop instruction to the automatic drainage device that requests stoppage of the drainage operation; and
a drainage operation stop step of stopping, in a case that the automatic drainage device receives the drainage stop instruction from the water quality management device, the drainage operation.
